# FASCICULE DE BREVET EUROPEEN

(11) **EP 4 313 222 B1**
(45) Date de publication et mention de la délivrance du brevet: **11.12.2024**
(21) Numéro de dépôt: 22717866.2
(22) Date de dépôt: 28.03.2022
(51) Int. Cl.: A61M 11/02, A61M 15/08, B05B 11/06

(54) **DISPOSITIF DE DISTRIBUTION NASALE DE POUDRE**
VORRICHTUNG ZUR NASALEN ABGABE VON PULVER
DEVICE FOR NASAL DELIVERY OF POWDER

(30) Priorité: 29.03.2021 FR 2103161
(43) Date de publication de la demande: 07.02.2024
(73) Titulaire: APTAR France SAS, 27110 Le Neubourg (FR)
(72) Inventeur: PETIT, Ludovic, 27110 VITOT (FR)
(74) Mandataire: CAPRI
(86) Numéro de dépôt international: PCT/FR2022/050576
(87) Numéro de publication internationale: WO 2022/208014

(56) Documents cités:
- EP-A1- 3 682 922
- EP-A2- 0 407 276
- WO-A1-2005/120617
- WO-A1-2007/141203
- WO-A1-2017/118827
- WO-A1-2019/220062
- US-A- 4 017 007
- US-A- 5 395 032

## Description

La présente invention concerne un dispositif de distribution nasale de poudre.

Les dispositifs de distribution nasale de poudre sont bien connus. Ils comportent généralement un réservoir contenant une ou plusieurs doses de poudre, des moyens de distribution, et une tête de distribution nasale destinée à être insérée dans une narine d'un utilisateur, ladite tête de distribution nasale comportant un orifice de distribution. Les moyens de distribution comportent généralement une chasse d'air. Lorsque le dispositif de distribution est actionné, une dose de poudre est distribuée dans une narine de l'utilisateur au moyen de l'écoulement d'air généré par la chasse d'air.

Un inconvénient avec ces dispositifs de l'art antérieur concerne la complexité du dispositif, notamment le nombre de pièces à fabriquer et à assembler.

Un autre inconvénient concerne la fiabilité du dispositif, qui peut dépendre fortement de la manière dont l'utilisateur actionne le dispositif. Ainsi, les performances de la chasse d'air peuvent être dépendantes de l'utilisateur, par exemple de la force et/ou de la vitesse d'actionnement, ce qui n'est pas toujours optimal.

Les documents US5395032, US4017007, EP3682922, EP0407276, EP0575239, WO9311818, WO9946055, WO0245866, WO2005120617, WO2007141203, WO2015001281, WO2017118827 et WO2019220062 décrivent des dispositifs de l'état de la technique.

La présente invention a pour but de fournir un dispositif de distribution nasale de poudre qui ne reproduit pas les inconvénients susmentionnés.

La présente invention a pour but de fournir un dispositif de distribution nasale de poudre qui est d'utilisation fiable.

La présente invention a également pour but de fournir un dispositif de distribution nasale de poudre dans lequel les performances de la chasse d'air sont substantiellement indépendantes de l'utilisateur et de sa manière d'actionner le dispositif.

La présente invention a aussi pour but de fournir un dispositif de distribution nasale de poudre qui est simple et peu coûteux à fabriquer et à assembler.

La présente invention a donc pour objet un dispositif de distribution nasale de poudre comportant un réservoir contenant une dose unique de poudre, une tête de distribution nasale comportant un orifice de distribution destinée à être insérée dans une narine d'un utilisateur, un organe d'actionnement déplaçable axialement par rapport à ladite tête lors de l'actionnement dudit dispositif, et une chasse d'air générant, lors de l'actionnement, un écoulement d'air comprimé pour distribuer ladite dose de poudre dans ladite narine à travers ledit orifice de distribution, ladite chasse d'air comprenant une chambre d'air, formée par un cylindre axial creux, et un piston d'air qui lors de l'actionnement coulisse de manière étanche dans ledit cylindre axial creux pour comprimer l'air contenu dans ladite chambre d'air, ledit dispositif comportant un insert formant ledit piston d'air et coopérant, en position de repos, de manière étanche avec ledit réservoir, ledit insert étant déplaçable axialement par rapport audit réservoir entre sa position de repos et une position d'actionnement, dans laquelle il ne coopère plus de manière étanche avec ledit réservoir, ledit réservoir comportant une partie cylindrique de ladite tête de distribution, ladite partie cylindrique comportant dans sa partie inférieure un évidement radial sur sa surface radialement interne et un piston sur sa surface radialement externe, ledit insert comportant un corps central pourvu d'un épaulement radial saillant radialement vers l'extérieur, ledit épaulement radial coopérant en position de repos de manière étanche avec une extrémité axiale inférieure de ladite partie cylindrique de ladite tête de distribution et coopérant en position d'actionnement avec ledit évidement radial.

Avantageusement, ledit dispositif comporte un capot amovible qui, en position de repos, ferme et protège ledit orifice de distribution et donc la dose de poudre contenue dans ledit réservoir.

Avantageusement, ledit cylindre axial creux de ladite chasse d'air est solidaire dudit organe d'actionnement monté coulissant par rapport à ladite tête de distribution.

Avantageusement, ledit cylindre creux de ladite chasse d'air comporte, au niveau d'une extrémité axiale inférieure, une découpe radiale formant une partie de cylindre de plus grand diamètre, de sorte que ledit piston d'air coopère de manière étanche avec ledit cylindre creux jusqu'à ce qu'il atteigne ladite découpe, où il ne coopère plus de manière étanche, permettant à l'air comprimé de s'échapper de ladite chambre d'air pour former ledit écoulement d'air comprimé utilisé pour expulser ladite dose de poudre dudit réservoir.

Avantageusement, ledit insert est déplacé de sa position de repos vers sa position d'actionnement en début de course d'actionnement, et ledit écoulement d'air comprimé est généré en fin de course d'actionnement.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique en section transversale d'un dispositif de distribution nasale de poudre selon un mode de réalisation avantageux, en position de repos avant actionnement,
la figure 2 est une vue similaire à celle de la figure 1, en début d'actionnement, et
la figure 3 est une vue similaire à celles des figures 1 et 2, en fin d'actionnement.

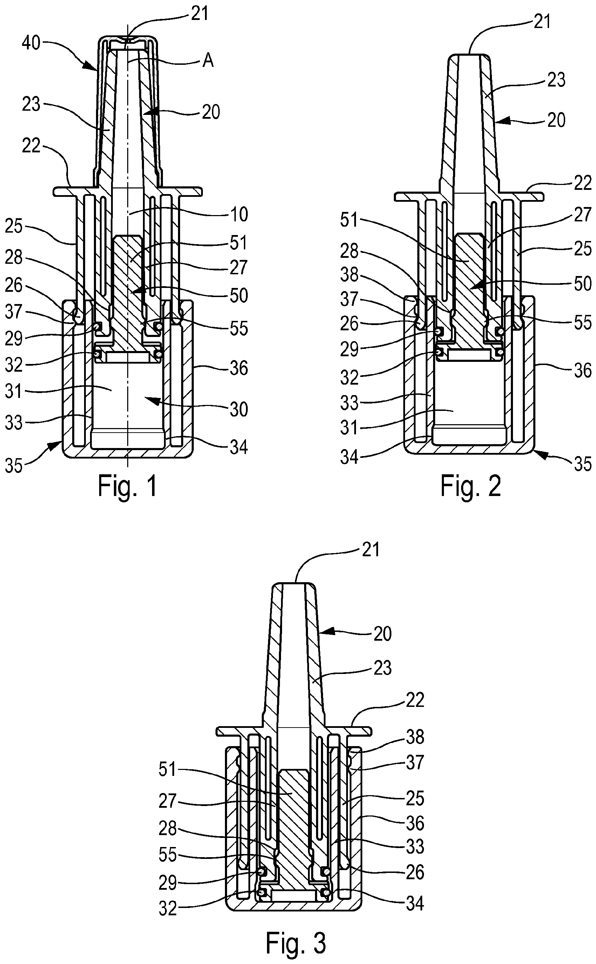

Dans la description, les termes "axial" et "radial" se réfèrent à l'axe longitudinale A du dispositif représenté sur la figure 1. Les termes "haut", "bas", "supérieur" et "inférieur" se réfèrent à la position droite du dispositif représentée sur les dessins.

L'invention s'applique à des dispositifs du type unidose poudre tel que celui représenté sur les figures. Bien entendu, d'autres types de dispositifs unidoses sont aussi envisageables.

Le dispositif représenté sur les figures comporte un réservoir 10 contenant une seule dose de poudre.

Une tête de distribution nasale 20 est associée au réservoir 10, ladite tête étant destinée à être insérée dans une narine d'un utilisateur. La tête de distribution nasale 20 comporte un orifice de distribution 21, de préférence orienté axialement. Elle comporte aussi avantageusement un repose-doigt 22 s'étendant radialement pour faciliter l'actionnement. Un embout nasal 23 s'étend axialement vers le haut à partir dudit repose-doigt 22 et se termine au niveau dudit orifice de distribution 21. Cet embout nasal 23 est de dimension radiale réduite pour pouvoir être inséré dans une narine au moment de l'actionnement. Du côté opposé du repose-doigt 22, une jupe 25 s'étend axialement vers le bas à partir dudit repose-doigt 22.

Le réservoir 10 est défini au moins en partie par une partie cylindrique 27 de la tête de distribution 20. Comme visible sur les dessins, cette partie cylindrique 27 est avantageusement disposée radialement à l'intérieur de la jupe 25 en lui étant coaxiale. Dans sa partie inférieure, la partie cylindrique 27 comporte sur sa surface radialement interne un évidement radial 28 et sur sa surface radialement externe un piston 29, dont les fonctions seront décrites ci-après. Dans l'exemple des figures, le piston 29 comporte un joint torique fixé dans un logement approprié, mais il pourrait être réalisée d'une quelconque manière appropriée, par exemple sous la forme bien connue d'une lèvre d'étanchéité.

Le dispositif comporte en outre une chasse d'air 30 générant, lors de l'actionnement, un écoulement d'air comprimé pour distribuer la dose de poudre dans ladite narine à travers l'orifice de distribution 21. La chasse d'air 30 comprend une chambre d'air 31 et un piston d'air 32 coulissant de manière étanche dans ladite chambre d'air 31 pour comprimer l'air contenu dans ladite chambre d'air 31 et ainsi générer ledit écoulement d'air comprimé.

La chambre d'air 31 est formée par un cylindre axial creux 33 qui est solidaire, de préférence monobloc, avec un organe d'actionnement 35 monté coulissant par rapport à la tête de distribution 20. Le côté axial inférieur dudit cylindre creux 33 est fermé et son côté supérieur est ouvert et obturé par ledit piston d'air 32.

Le cylindre creux 33 comporte au niveau de son extrémité axiale inférieure une découpe radiale 34 qui forme une partie de cylindre de plus grand diamètre. Ainsi, le piston d'air 32 coopère de manière étanche avec le cylindre creux 33 jusqu'à ce qu'il atteigne ladite découpe 34, où il ne coopère plus de manière étanche, permettant à l'air comprimé de s'échapper pour former l'écoulement d'air comprimé de la chasse d'air utilisé pour expulser la dose de poudre du réservoir.

L'organe d'actionnement 35 comporte un manchon externe 36 coaxial audit cylindre creux 33 et définissant un espace entre eux.

La jupe 25 est avantageusement disposée coulissante dans l'espace défini entre ledit cylindre creux 33 et ledit manchon externe 36. Dans cette mise en oeuvre, le manchon externe 36 et/ou le cylindre creux 33 peut/peuvent comporter des moyens de précompression 37, tel qu'une ou plusieurs projection(s) radiale(s), coopérant avec ladite jupe 25. Ces moyens de précompression 37 nécessitent pour être surmontés que l'utilisateur exerce une force d'actionnement prédéterminée. Avantageusement, la jupe 25 comporte à son extrémité axiale inférieure un profil radial 26 coopérant avec lesdits moyens de précompression 37 en tout début d'actionnement. Typiquement, à partir d'une force d'actionnement prédéterminée, le profil radial 26 de la jupe 25 peut passer par-dessus la projection radiale 37. Une telle précompression dans la main de l'utilisateur brusquement libérée garantit que l'actionnement sera réalisé de manière complète, sans risque d'un actionnement partiel.

Avantageusement, le manchon externe 36 et/ou le cylindre creux 33 peut/peuvent comporter des moyens de retenue 38, tel qu'une ou plusieurs projection(s) radiale(s), pour assurer le maintien de la tête de distribution 20 sur l'organe d'actionnement 35.

En positon de repos, le profil radial 26 de la jupe 25 est avantageusement coincé entre les projections 37 et 38 de l'organe d'actionnement 35.

Dans l'exemple représenté sur les figures, le réservoir 10 est formé à l'intérieur de la tête de distribution 20, et est ouvert à ses deux extrémités axiales. En position de repos, le réservoir 10 est fermé au niveau de son extrémité axiale supérieure par un capot amovible 40, visible sur la figure 1. Ce capot 40 sert à fermer et protéger l'orifice de distribution 21 et le réservoir 10, et donc la dose de poudre qu'il contient. L'extrémité axiale inférieure du réservoir 10 est fermée par un insert 50.

Cet insert 50 comporte un corps central 51 pourvu d'un épaulement radial 55 saillant radialement vers l'extérieur. L'épaulement radial 55 coopère en position de repos de manière étanche avec l'extrémité axiale inférieure de la partie cylindrique 27 de la tête de distribution 20. En cours d'actionnement, l'insert 50 est légèrement déplacé axialement vers le haut par rapport à la tête 20 vers sa position d'actionnement, dans laquelle l'épaulement radial 55 coopère alors avec l'évidement radial 28. Dans cette position d'actionnement, l'écoulement d'air généré par la chasse d'air 30 peut alors passer autour dudit épaulement radial 55 et entrainer avec lui la poudre contenue dans le réservoir 10.

Le piston d'air 32 de la chasse d'air 30 est avantageusement formé sur ledit insert 50.

Dans l'exemple des figures, le piston d'air 32 comporte un joint torique fixé dans un logement approprié dudit insert 50, mais il pourrait être réalisée d'une quelconque manière appropriée, par exemple sous la forme bien connue d'une lèvre d'étanchéité.

Le fonctionnement du dispositif de distribution va maintenant être décrit en référence aux figures.

En position de repos, visible sur la figure 1, la dose de poudre disposée dans le réservoir 10 est protégée d'une part par le capot amovible 40 positionné sur la tête 20 et d'autre part par l'insert 50 en position d'obturation étanche par rapport à la tête 20.

Lorsqu'un utilisateur souhaite utiliser le dispositif, il retire le capot amovible 40 et insère l'embout nasal 23 de la tête 20 dans une narine.

Pour actionner le dispositif, il place ses doigts sur le repose-doigts 22 de la tête 20 et le pouce sur le fond de l'organe d'actionnement 35, et exerce une force d'actionnement axiale entre ses doigts et son pouce.

Les moyens de précompression 37 obligent l'utilisateur à exercer une force prédéterminée pour déclencher l'actionnement.

Lorsque cette force prédéterminée est exercée, la tête 20 peut se déplacer axialement vers le bas par rapport à l'organe d'actionnement 35. La résistance exercée par la chambre d'air 31 sur le piston d'air 32 étant supérieure à celle exercée par la tête 20 sur l'insert 50, en début d'actionnement l'insert 50 ne bouge pas par rapport à l'organe d'actionnement 35, et c'est la tête qui se déplace légèrement par rapport à l'organe d'actionnement 35 et par rapport à l'insert 50. Ceci a pour effet de déplacer l'insert 50 de sa position d'obturation étanche dans sa position d'actionnement dans laquelle l'épaulement radial 55 est disposé dans l'évidement radial 28, comme visible sur la figure 2.

Dans cette position, l'extrémité axiale inférieure de la partie cylindrique 27 de la tête 20 vient en contact avec le piston d'air 32, et une poursuite du déplacement axial de la tête 20 par rapport à l'organe d'actionnement 35 va donc déplacer axialement l'insert 50 par rapport à l'organe d'actionnement 35, avec par conséquent un déplacement du piston d'air 32 dans la chambre d'air 31, ce qui comprime l'air contenu dans la chambre d'air.

Lorsque le piston d'air 32 atteint la découpe radiale 34, en fin de course d'actionnement, l'air comprimé peut s'échapper de la chambre d'air 31 pour aller expulser la dose de poudre contenue dans le réservoir 10.

La présente invention permet donc de réaliser un dispositif de distribution unidose de poudre avec seulement trois pièces constitutives, à savoir la tête de distribution 20, l'organe d'actionnement 35 et l'insert 50, et une pièce amovible, à savoir le capot 40. La fabrication et l'assemblage du dispositif sont donc particulièrement simples et peu coûteux.

De plus, la chasse d'air est indépendante de l'utilisateur, car quelle que soit la force ou la vitesse d'actionnement, l'écoulement d'air comprimé n'est libéré qu'en fin de course d'actionnement, avec donc toujours les mêmes performances, notamment en termes de pression et de débit.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution nasale de poudre comportant un réservoir (10) contenant une dose unique de poudre, une tête de distribution nasale (20) comportant un orifice de distribution (21) destinée à être insérée dans une narine d'un utilisateur, un organe d'actionnement (35) déplaçable axialement par rapport à ladite tête (20) lors de l'actionnement dudit dispositif, et une chasse d'air (30) générant, lors de l'actionnement, un écoulement d'air comprimé pour distribuer ladite dose de poudre dans ladite narine à travers ledit orifice de distribution (21), ladite chasse d'air comprenant une chambre d'air (31), formée par un cylindre axial creux (33), et un piston d'air (32) qui lors de l'actionnement coulisse de manière étanche dans ledit cylindre axial creux (33) pour comprimer l'air contenu dans ladite chambre d'air (31), **caractérisé en ce que** ledit dispositif comporte un insert (50) formant ledit piston d'air (32) et coopérant, en position de repos, de manière étanche avec ledit réservoir (10), ledit insert (50) étant déplaçable axialement par rapport audit réservoir (10) entre sa position de repos et une position d'actionnement, dans laquelle il ne coopère plus de manière étanche avec ledit réservoir (10), ledit réservoir (10) comportant une partie cylindrique (27) de ladite tête de distribution (20), ladite partie cylindrique (27) comportant dans sa partie inférieure un évidement radial (28) sur sa surface radialement interne et un piston (29) sur sa surface radialement externe, ledit insert (50) comportant un corps central (51) pourvu d'un épaulement radial (55) saillant radialement vers l'extérieur, ledit épaulement radial (55) coopérant en position de repos de manière étanche avec une extrémité axiale inférieure de ladite partie cylindrique (27) de ladite tête de distribution (20) et coopérant en position d'actionnement avec ledit évidement radial (28).

2. Dispositif selon la revendication 1, comportant un capot amovible (40) qui, en position de repos, ferme et protège ledit orifice de distribution (21) et donc la dose de poudre contenue dans ledit réservoir (10).

3. Dispositif selon la revendication 1 ou 2, dans lequel ledit cylindre axial creux (33) de ladite chasse d'air (30) est solidaire dudit organe d'actionnement (35) monté coulissant par rapport à ladite tête de distribution (20).

4. Dispositif selon l'une quelconque des revendications précédentes, dans lequel ledit cylindre creux (33) de ladite chasse d'air (30) comporte, au niveau d'une extrémité axiale inférieure, une découpe radiale (34) formant une partie de cylindre de plus grand diamètre, de sorte que ledit piston d'air (32) coopère de manière étanche avec ledit cylindre creux (33) jusqu'à ce qu'il atteigne ladite découpe (34), où il ne coopère plus de manière étanche, permettant à l'air comprimé de s'échapper de ladite chambre d'air (31) pour former ledit écoulement d'air comprimé utilisé pour expulser ladite dose de poudre dudit réservoir (10).

5. Dispositif selon l'une quelconque des revendications précédentes, dans lequel ledit insert (50) est déplacé de sa position de repos vers sa position d'actionnement en début de déplacement dudit organe d'actionnement (35), et ledit écoulement d'air comprimé est généré en fin de déplacement dudit organe d'actionnement (35).

## Patentansprüche

1. Vorrichtung zur nasalen Abgabe von Pulver, mit einem Vorratsbehälter (10) mit darin enthaltener Pulver-Einzeldosis, einem Kopf (20) zur nasalen Abgabe mit Abgabeöffnung (21) zur Einführung in ein Nasenloch eines Benutzers, einem bei Betätigung der Vorrichtung in Bezug auf den Kopf (20) axial verschiebbaren Betätigungsorgan (35), und einer Luftausstoßvorrichtung (30), die bei Betätigung einen Druckluftstrom zur Abgabe der Pulverdosis über die Abgabeöffnung (21) in das Nasenloch erzeugt, wobei die Luftausstoßvorrichtung eine durch einen hohlen Axialzylinder (33) gebildete Luftkammer (31) und einen Luftkolben (32) umfasst, der bei Betätigung abdichtend in dem hohlen Axialzylinder (33) gleitet und dabei die in der Luftkammer (31) enthaltene Luft komprimiert, **dadurch gekennzeichnet, dass** die Vorrichtung einen Einsatz (50) aufweist, der den Luftkolben (32) bildet und in der Ruhestellung abdichtend mit dem Vorratsbehälter (10) zusammenwirkt, wobei der Einsatz (50) zwischen seiner Ruhestellung und einer Betätigungsstellung, in der er nicht mehr abdichtend mit dem Vorratsbehälter (10) zusammenwirkt, in Bezug auf den Vorratsbehälter (10) axial verschiebbar ist, wobei der Vorratsbehälter (10) einen zylindrischen Abschnitt (27) des Abgabekopfes (20) aufweist, wobei der zylindrische Abschnitt (27) in seinem unteren Abschnitt eine radiale Ausnehmung (28) an seiner radial inneren Oberfläche und einen Kolben (29) an seiner radial äußeren Oberfläche aufweist, wobei der Einsatz (50) einen zentralen Körper (51) mit einer radial nach außen vorstehenden radialen Schulter (55) aufweist, wobei die radiale Schulter (55) in der Ruhestellung abdichtend mit einem unteren axialen Ende des zylindrischen Abschnitts (27) des Abgabekopfes (20) zusammenwirkt und in der Betätigungsstellung mit der radialen Ausnehmung (28) zusammenwirkt.

2. Vorrichtung nach Anspruch 1, umfassend eine abnehmbare Kappe (40), die in der Ruhestellung die Abgabeöffnung (21), und entsprechend auch die in dem Vorratsbehälter (10) enthaltene Pulverdosis, verschließt und schützt.

3. Vorrichtung nach Anspruch 1 oder 2, bei der der hohle Axialzylinder (33) der Luftausstoßvorrichtung (30) fest mit dem Betätigungsorgan (35) verbunden ist, welches in Bezug auf den Abgabekopf (20) verschiebbar gelagert ist.

4. Vorrichtung nach einem der vorhergehenden Ansprüche, bei der der Hohlzylinder (33) der Luftausstoßvorrichtung (30) an einem unteren axialen Ende eine radiale Aussparung (34) aufweist, die einen Zylinderteil mit größerem Durchmesser bildet, sodass der Luftkolben (32) abdichtend mit dem Hohlzylinder (33) zusammenwirkt, bis er die Aussparung (34) erreicht, an der er nicht mehr abdichtend zusammenwirkt, wodurch die Druckluft aus der Luftkammer (31) entweichen und den zum Ausstoß der Pulverdosis aus dem Vorratsbehälter (10) verwendeten Druckluftstrom bilden kann.

5. Vorrichtung nach einem der vorhergehenden Ansprüche, bei der der Einsatz (50) zu Beginn der Bewegung des Betätigungsorgans (35) aus seiner Ruheposition in seine Betätigungsposition verschoben wird und die Erzeugung des Druckluftstroms am Ende der Bewegung des Betätigungsorgans (35) erfolgt.

## Claims

1. Device for nasal delivery of powder having a reservoir (10) containing one single dose of powder, a nasal delivery head (20) having a delivery orifice (21) intended to be inserted into a nostril of a user, an actuating member (35) axially displaceable with respect to said head (20) during the actuation of said device, and an air expeller (30) generating, during the actuation, a flow of compressed air to deliver said dose of powder into said nostril through said delivery orifice (21), said air expeller comprising an air chamber (31), formed by a hollow axial cylinder (33), and an air piston (32), which during actuation, slides sealingly into said hollow axial cylinder (33) to compress the air contained in said air chamber (31), **characterised in that** said device comprises an insert (50) forming said air piston (32) and interacting sealingly in a rest position with said reservoir (10), said insert (50) being axially displaceable with respect to said reservoir (10) between its rest position and an actuation position, in which it no longer interacts sealingly with said reservoir (10), said reservoir (10), comprising a cylindrical part (27) of said delivery head (20), said cylindrical part (27) having in its lower part, a radial recess (28) on its radially internal surface and a piston (29) on its radially external surface, said insert (50) having a central body (51) provided with a radial shoulder (55) projecting radially outwards, said radial shoulder (55) interacting sealingly in a rest position with a lower axial end of said cylindrical part (27) of said delivery head (20) and interacting in an actuation position with said radial recess (28).

2. Device according to claim 1, comprising a removable cap (40) which, in a rest position, closes and protects said delivery orifice (21) and therefore the dose of powder contained in said reservoir (10).

3. Device according to claim 1 or 2, wherein said hollow axial cylinder (33) of said air expeller (30) is integral with said actuating member (35) slidingly mounted with respect to said delivery head (20).

4. Device according to any one of the preceding claims, wherein said hollow cylinder (33) of said air expeller (30) has, at a lower axial end, a radial cutout (34) forming a cylinder part of a greater diameter, such that said air piston (32) interacts sealingly with said hollow cylinder (33) until it reaches said cutout (34), where it no longer interacts sealingly, enabling the compressed air to be discharged from said air chamber (31) to form said flow of compressed air used to expel said dose of powder from said reservoir (10).

5. Device according to any one of the preceding claims, wherein said insert (50) is displaced from its rest position to its actuation position at the start of the displacement of said actuating member (35), and said flow of compressed air is generated at the end of the displacement of said actuating member (35).
